# EUROPEAN PATENT APPLICATION

(11) **EP 4 197 591 A1**
(43) Date of publication of application: **21.06.2023**
(21) Application number: 21855793.2
(22) Date of filing: 13.04.2021
(51) Int. Cl.: A61N 5/10

(54) **PARTICLE BEAM IRRADIATION SYSTEM AND CONTROL METHOD THEREFOR, AND CONTROL DEVICE FOR PARTICLE BEAM IRRADIATION SYSTEM**

(30) Priority: 11.08.2020 JP 2020135881
(71) Applicant: Hitachi, Ltd., Tokyo 100-8280 (JP)
(72) Inventor: TAKAHASHI Kenta, Tokyo 100-8280 (JP); OKURA Satoru, Tokyo 100-8280 (JP); ITO Yuki, Tokyo 100-8280 (JP); YAMADA Kohei, Tokyo 100-8280 (JP)
(74) Representative: MERH-IP Matias Erny Reichl Hoffmann Patentanwälte PartG mbB
(86) International application number: PCT/JP2021/015301
(87) International publication number: WO 2022/034714

(57) **Abstract**

In order to prevent an unexpected suspension of treatment on a condition that sufficient safety is maintained, provided is a particle beam irradiation system including: a charged particle beam generation unit; an irradiation unit including an irradiation dose monitor and configured to provide spot-by-spot irradiation at an object to be irradiated with a charged particle beam, the spot-by-spot irradiation performed by sequentially providing irradiation of the charged particle beam at a plurality of spots one by one in the object; a scanning controller; and an accelerator and transport system controller. In the particle beam irradiation system, when, on receipt of a signal to stop the irradiation of the charged particle beam, the signal outputted from the scanning controller, the accelerator and transport system controller stops emission of the charged particle beam from the charged particle beam generation unit to the irradiation unit, the scanning controller determines, in accordance with an irradiation dose of the charged particle beam at one of the plurality of spots that has been irradiated with the charged particle beam until immediately before the accelerator and transport system controller stops the emission, the irradiation dose measured by the irradiation dose monitor from when the signal to stop the irradiation is outputted, whether or not to skip the irradiation of the charged particle beam at another one of the plurality of spots subsequent to the one of the plurality of spots, so as to control the accelerator and transport system controller.

## Description

### Technical Field

The present invention relates to a particle beam irradiation system, a control method for the particle beam irradiation system, and a control device for the particle beam irradiation system.

### Background Art

A particle beam irradiation system is configured to control spot-by-spot emission of a particle beam (hereinafter, may also simply be referred to as a beam) to conduct a treatment. In recent years, in response to a demand for irradiation at a further complex shape and a further complex irradiation dose distribution of an object to be irradiated, a spot-by-spot irradiation dose has tended to be reduced, so that the particle beam irradiation system has been required to have a higher level of control function to continue with the treatment even when some spots require the reduced irradiation dose.

PTL 1 (JP 3806723 B2) provides a "particle beam irradiation system" configured, instead of spot-by-spot monitoring of the irradiation dose, to monitor a cumulative irradiation dose of each of the spots, so as to provide a more uniformed irradiation dose distribution of the object to be irradiated.

### Citation List

### Patent Literature

PTL 1: JP 3806723 B2

### Summary of Invention

### Technical Problem

In an embodiment of the particle beam irradiation system according to PTL 1, when a signal to start emission of the beam at each of the spots is outputted, and when the irradiation dose, which is measured during a period from when a target irradiation dose for a spot prior to the corresponding spot is achieved until when the signal is outputted, has already exceeded a target irradiation dose for the corresponding spot, an error occurs. This configuration causes suspension of the irradiation, making it impossible to continue with the treatment.

Here, the irradiation dose is measured in a situation where an accelerator of the particle beam irradiation system has a response delay, and thus a measured value of the irradiation dose varies in accordance with a beam current value. Accordingly, when the beam current value unexpectedly increases, the irradiation dose rapidly increases, which may lead to unexpected suspension of the treatment.

Further, due to the response delay, even when the irradiation dose measured is still below the target irradiation dose for the corresponding spot, the irradiation dose may be extremely small. At such a spot, the actual irradiation dose is reduced too, possibly causing the monitoring function to detect a "set value deviation" error with the actual irradiation dose measured after the irradiation, and thus causing the suspension of the treatment.

Such an issue is prone to arise particularly at a spot requiring a small irradiation dose.

The present invention has been developed to solve the problems described above, and an object of the present invention is, on a condition that sufficient safety is maintained, to provide a particle beam irradiation system including a "skip-spot function" so as to prevent the unexpected suspension of the treatment, and to provide a control method for the particle beam irradiation system as well as a control device for the particle beam irradiation system.

### Solution to Problem

In order to achieve the object, the present invention provides a particle beam irradiation system including: a charged particle beam generation unit configured to generate a charged particle beam; an irradiation unit including a scanning electromagnet configured to provide spot-by-spot irradiation at an object to be irradiated with the charged particle beam generated by the charged particle beam generation unit, the spot-by-spot irradiation performed by sequentially providing irradiation of the charged particle beam at a plurality of spots one by one in the object, and an irradiation dose monitor configured to measure an irradiation dose of the charged particle beam; a scanning controller configured to generate a signal to start or stop the irradiation of the charged particle beam provided by the irradiation unit at the object to be irradiated; and an accelerator and transport system controller configured, on receipt of the signal to start or stop the irradiation of the charged particle beam outputted from the scanning controller, to start or stop emission of the charged particle beam from the charged particle beam generation unit to the irradiation unit. In the particle beam irradiation system, when, on the receipt of the signal to stop the irradiation of the charged particle beam, the accelerator and transport system controller stops the emission of the charged particle beam from the charged particle beam generation unit to the irradiation unit, the scanning controller determines, in accordance with the irradiation dose at one of the plurality of spots that has been irradiated with the charged particle beam until immediately before the accelerator and transport system controller stops the emission, the irradiation dose measured by the irradiation dose monitor from when the signal to stop the irradiation is outputted from the scanning controller, whether or not to skip the irradiation of the charged particle beam at another one of the plurality of spots subsequent to the one of the plurality of spots, so as to control the accelerator and transport system controller.

In order to achieve the object, the present invention provides a particle beam irradiation system including: a charged particle beam generation unit configured to generate a charged particle beam; an irradiation unit including a scanning electromagnet configured to provide spot-by-spot irradiation at an object to be irradiated with the charged particle beam generated by the charged particle beam generation unit, the spot-by-spot irradiation performed by sequentially and consecutively providing irradiation of the charged particle beam at a plurality of spots in the object, and an irradiation dose monitor configured to measure an irradiation dose of the charged particle beam; a scanning controller configured to generate a signal to start or stop the irradiation of the charged particle beam provided by the irradiation unit to the object to be irradiated; and an accelerator and transport system controller configured, on receipt of the signal to start or stop the irradiation of the charged particle beam outputted from the scanning controller, to start or stop emission of the charged particle beam from the charged particle beam generation unit to the irradiation unit. In the particle beam irradiation system, the scanning controller controls the accelerator and transport system controller to consecutively provide the irradiation of the charged particle beam at the plurality of spots, the scanning controller outputs to the accelerator and transport system controller the signal to stop the irradiation of the charged particle beam when a cumulative total of the irradiation dose of the charged particle beam consecutively provided at each of the plurality of spots, the irradiation dose measured by the irradiation dose monitor, has reached a predetermined irradiation dose, and the scanning controller determines, in accordance with the irradiation dose measured by the irradiation dose monitor from when the signal to stop the irradiation is outputted, whether or not to skip the irradiation of the charged particle beam at a first one of a plurality of spots that are to be consecutively irradiated subsequent to the plurality of spots that have been consecutively irradiated with the charged particle beam, so as to control the accelerator and transport system controller.

In order to achieve the object, the present invention provides a control method for a particle beam irradiation system including a charged particle beam generation unit configured to generate a charged particle beam, an irradiation unit configured to sequentially provide irradiation of the charged particle beam at a plurality of spots one by one in an object to be irradiated, the irradiation unit including an irradiation dose monitor to measure an irradiation dose of the charged particle beam at the plurality of spots, a scanning controller configured to output a signal to start or stop the irradiation of the charged particle beam, and an accelerator and transport system controller configured, based on the signal outputted from the scanning controller, to start or stop the irradiation of the charged particle beam. The control method is performed by the scanning controller and includes: outputting to the accelerator and transport system controller the signal to stop the irradiation of the charged particle beam when the irradiation dose of the charged particle beam at one of the plurality of spots that has been irradiated with the charged particle beam, the irradiation dose measured by the irradiation dose monitor, has reached a predetermined irradiation dose; and determining, in accordance with the irradiation dose measured at the one of the plurality of spots by the irradiation dose monitor from when the signal to stop the irradiation is outputted, whether or not to skip the irradiation of the charged particle beam at another one of the plurality of spots subsequent to the one of the plurality of spots, so as to control the accelerator and transport system controller.

In order to achieve the object, the present invention provides a control method for a particle beam irradiation system including a charged particle beam generation unit configured to generate a charged particle beam, an irradiation unit configured to consecutively provide irradiation of the charged particle beam at a plurality of spots in an object to be irradiated, the irradiation unit including an irradiation dose monitor to measure an irradiation dose of the charged particle beam at the plurality of spots, a scanning controller configured to output a signal to start or stop the irradiation of the charged particle beam, and an accelerator and transport system controller configured, based on the signal outputted from the scanning controller, to start or stop the irradiation of the charged particle beam. The control method is performed by the scanning controller and includes: outputting to the accelerator and transport system controller the signal to stop the irradiation of the charged particle beam when a cumulative total of the irradiation dose of the charged particle beam consecutively provided at each of the plurality of spots, the irradiation dose measured by the irradiation dose monitor, has reached a predetermined irradiation dose, and determining, in accordance with the irradiation dose measured by the irradiation dose monitor from when the signal to stop the irradiation is outputted, whether or not to skip the irradiation of the charged particle beam at a first one of a plurality of spots that are to be consecutively irradiated subsequent to the plurality of spots that have been consecutively irradiated with the charged particle beam, so as to control the accelerator and transport system controller.

In order to achieve the object, the present invention provides a control device for a particle beam irradiation system including a charged particle beam generation unit configured to generate a charged particle beam, and an irradiation unit configured to provide spot-by-spot irradiation at an object to be irradiated with the charged particle beam generated by the charged particle beam generation unit, the spot-by-spot irradiation performed by sequentially providing irradiation of the charged particle beam at a plurality of spots one by one in the object. The control device includes: a scanning controller configured to generate a signal to start or stop the irradiation of the charged particle beam provided by the irradiation unit at the object to be irradiated; and an accelerator and transport system controller configured, on receipt of the signal to stop the irradiation of the charged particle beam outputted from the scanning controller, to start or stop emission of the charged particle beam from the charged particle beam generation unit to the irradiation unit. In the control device, when an irradiation dose of the charged particle beam at one of the plurality of spots that has been irradiated with the charged particle beam, the irradiation dose measured by an irradiation dose monitor, has reached a predetermined irradiation dose, the scanning controller outputs to the accelerator and transport system controller the signal to stop the irradiation of the charged particle beam, and the scanning controller determines, in accordance with the irradiation dose measured by the irradiation dose monitor at the one of the plurality of spots from when the signal to stop the irradiation is outputted, whether or not to skip the irradiation of the charged particle beam at another one of the plurality of spots subsequent to the one of the plurality of spots, so as to control the accelerator and transport system controller.

In order to achieve the object, the present invention provides a control device for a particle beam irradiation system including a charged particle beam generation unit configured to generate a charged particle beam, an irradiation unit configured to consecutively provide irradiation of the charged particle beam generated by the charged particle beam generation unit at a plurality of spots in an object to be irradiated, and an irradiation dose monitor configured to monitor and measure an irradiation dose of the charged particle beam provided by the irradiation unit. The control device includes: a scanning controller configured to generate a signal to start or stop the irradiation of the charged particle beam provided by the irradiation unit at the object to be irradiated; and an accelerator and transport system controller configured, on receipt of the signal to stop the irradiation of the charged particle beam outputted from the scanning controller, to start or stop emission of the charged particle beam from the charged particle beam generation unit to the irradiation unit. In the control device, the scanning controller outputs to the accelerator and transport system controller the signal to stop the irradiation of the charged particle beam when a cumulative total of the irradiation dose of the charged particle beam consecutively provided at each of the plurality of spots, the irradiation dose measured by the irradiation dose monitor, has reached a predetermined irradiation dose, and the scanning controller determines, in accordance with the irradiation dose measured by the irradiation dose monitor from when the signal to stop the irradiation is outputted, whether or not to skip the irradiation of the charged particle beam at a first one of a plurality of spots that are to be consecutively irradiated subsequent to the plurality of spots that have been consecutively irradiated with the charged particle beam, so as to control the accelerator and transport system controller.

### Advantageous Effects of Invention

The present invention provides a particle beam irradiation system, a control method for the particle beam irradiation system, and a control device for the particle beam irradiation system, each configured, on a condition that sufficient safety is maintained, to fulfil a skip function so as to continue with a treatment, even when, at time of output of a signal to start emission of a particle beam at a spot, an irradiation dose of the particle beam at the spot has already exceeded a target irradiation dose for the spot and the target irradiation dose is thus zero, or even when the spot requires a small irradiation dose due to a response delay of an accelerator, a complex shape, or a complex irradiation dose distribution.

### Brief Description of Drawings

[FIG. 1] FIG. 1 is a block diagram illustrating an overall configuration of a particle beam irradiation system according to a first embodiment and a second embodiment of the present invention.
[FIG. 2] FIG. 2 is a flowchart showing a control procedure executed by a scanning controller and an accelerator and transport system controller in the first embodiment.
[FIG. 3] FIG. 3 is a flowchart showing details of the control procedure executed by the scanning controller in the first embodiment.
[FIG. 4] FIG. 4 is a graph showing a transition of an irradiation dose at each spot, the irradiation dose achieved by the control procedure of the scanning controller in the first embodiment.
[FIG. 5] FIG. 5 is a flowchart showing a control procedure executed by the scanning controller and the accelerator and transport system controller in the second embodiment.
[FIG. 6] FIG. 6 is a flowchart showing details of the control procedure executed by the scanning controller in the second embodiment.
[FIG. 7] FIG. 7 is a graph showing a transition of an irradiation dose at each spot, the irradiation dose achieved by the control procedure of the scanning controller in the second embodiment.

### Description of Embodiments

The present invention provides a particle beam irradiation system configured, in a case that when a signal to start emission of a particle beam to a spot is outputted, an irradiation dose of the particle beam at the spot has already exceeded a target irradiation dose for the spot, to skip the emission of the particle beam at the spot and proceed to a spot subsequent to the spot.

Additionally, the particle beam irradiation system is configured, in a case where the irradiation dose of the particle beam at the spot is equal to or smaller than a lower limit value that is predetermined before the irradiation of the particle beam starts, to skip determination on an actual irradiation dose at the spot and proceed to a spot subsequent to the spot. Note that, with regard to skipping the determination on the actual irradiation dose, the particle beam irradiation system has a monitoring function such that safety of treatment is maintained.

An embodiment of the present invention will be described in detail below with reference to the drawings. In the drawings for describing this embodiment, same names and reference signs represent identical or equivalent components or functions, and a detailed description thereof will be omitted as appropriate.

Note that, the present invention is not limited to the embodiments below. Accordingly, it is easily understood for those skilled in the art that any change, addition, or deletion of a configuration of each unit appropriately made within the spirit of the present invention will naturally fall within the scope of claims of the present invention.

### First embodiment

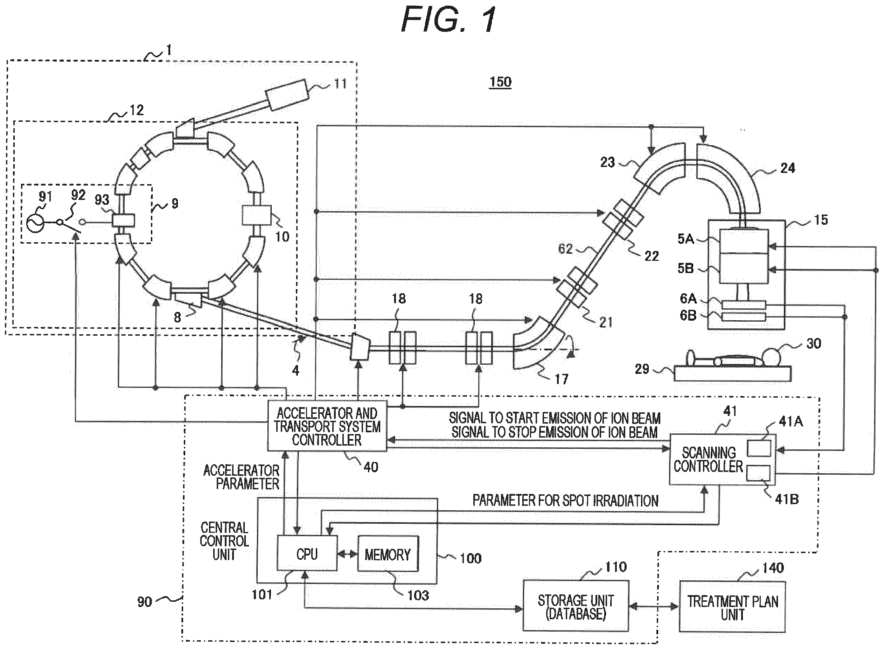

FIG. 1 illustrates a particle beam irradiation system 150 of this embodiment.

The particle beam irradiation system 150 of this embodiment includes a charged particle beam generation unit 1, and a beam transport system 4 connected to a downstream side of the charged particle beam generation unit 1.

The charged particle beam generation unit 1 includes an ion source (not illustrated), a pre-stage charged particle beam generation unit (LINAC) 11, and a synchrotron (accelerator) 12. The synchrotron 12 includes a radio-frequency applicator 9 and an accelerator mechanism 10.

The radio-frequency applicator 9 includes a radio-frequency applicator electrode 93 arranged in a circling orbit of the synchrotron 12, a radio-frequency power supply 91, and an opening/closing switch 92 configured to connect the radio-frequency applicator electrode 93 with the radio-frequency power supply 91.

The accelerator mechanism (a second element or a charged particle beam energy change mechanism) 10 includes a radio-frequency accelerator cavity (not illustrated) arranged in the circular orbit, and a radio-frequency power supply (not illustrated) configured to apply radio-frequency power to the radio-frequency accelerator cavity.

An ion (e.g., proton ions (or carbon ions)) generated by the ion source (not illustrated) is accelerated by the pre-stage charged particle beam generation unit (e.g., a linear charged particle beam generation unit) 11.

The pre-stage charged particle beam generation unit 11 emits an ion beam to be incident on the synchrotron 12. In the synchrotron 12, the ion beam as a charged particle beam (particle beam) is accelerated by being energized by the radio-frequency power that is applied from the radio-frequency power supply of the accelerator mechanism 10. Here, the radio-frequency power is applied to the ion beam through the radio-frequency accelerator cavity of the accelerator mechanism 10. When the ion beam has been increased to an energy level predetermined, the radio-frequency power for emission applied from the radio-frequency power supply 91 travels, through the opening/closing switch 92 in a closed state, to the radio-frequency applicator electrode 93 in the radio-frequency applicator 9; and then the radio-frequency power for emission is applied from the radio-frequency applicator electrode 93 to the ion beam.

When the radio-frequency power is applied from the radio-frequency applicator electrode 93 to the ion beam circulating within a stability limit, the ion beam moves out of the stability limit, and is emitted from the synchrotron 12, through an emission deflector 8, to the beam transport system 4.

Here, when the opening/closing switch 92 is opened to turn off the radio-frequency power applied to the radio-frequency applicator electrode 93, the emission of the ion beam from the synchrotron 12 stops. With the synchrotron 12 as the accelerator of this embodiment, even when an accelerator and transport system controller (as will be described later) inputs a command to stop the emission of the ion beam, the synchrotron 12 has features that may cause its response to be somewhat delayed, strictly speaking, until the opening/closing switch 92 of the radio-frequency applicator 9 is opened and the emission of the ion beam through the emission deflector 8 to the beam transport system 4 actually stops.

The ion beam emitted from the synchrotron 12 is transported through the emission deflector 8 to the downstream side of the beam transport system 4. The beam transport system 4 includes a quadrupole electromagnet 18, a deflection electromagnet 17, a beam path 62, a quadrupole electromagnet 21, a quadrupole electromagnet 22, a deflection electromagnet 23, and a deflection electromagnet 24 (each electromagnet corresponding to a first element). The beam path 62 communicates to an irradiation unit 15 arranged in a treatment room (not illustrated), and the quadrupole electromagnet 21, the quadrupole electromagnet 22, the deflection electromagnet 23, and the deflection electromagnet 24 are arranged on the beam path 62, sequentially from an upstream side in a direction where the ion beam travels.

The ion beam introduced into the beam transport system 4 is transported through the beam path 62 to the irradiation unit 15 of the treatment room (not illustrated).

The treatment room has a rotary gantry (not illustrated) disposed therein, and includes the irradiation unit 15 attached to the rotary gantry. The rotary gantry (not illustrated) has a rotary drum (not illustrated) of substantially cylindrical shape, and the rotary drum has the irradiation unit 15 and a beam transport unit disposed thereon. The beam transport unit is inverted U-shaped and includes a part of the beam path 62 of the beam transport system 4. The rotary drum is rotatable by a motor (not illustrated). The rotary drum has a treatment gauge (not illustrated) arranged therein.

The irradiation unit 15 includes a casing (not illustrated) attached to the rotary drum and connected to the beam transport unit of the inverted U-shape as described above. The casing has a scanning electromagnet 5A and a scanning electromagnet 5B, each configured to scan the ion beam, together with an irradiation dose monitor 6A and a position monitor 6B.

When the ion beam has been introduced from the beam transport unit of the inverted U-shape into the irradiation unit 15 through the beam path 62, the scanning electromagnets (charged particle beam scanners) 5A and 5B sequentially and two-dimensionally scan positions to be irradiated with the ion beam, so that a patient 30 lying on a treatment bed 29 has his/her affected area (e.g., a cancer or tumor affected area) to be irradiated with the ion beam. The ion beam emits its energy at the affected areas to form a high irradiation dose area.

The particle beam irradiation system 150 of this embodiment has a control system 90 that includes a central control unit 100, a storage unit 110, a scanning controller 41, and an accelerator and transport system controller (hereinafter, referred to as an accelerator controller) 40. The storage unit 110 stores treatment plan information. The particle beam irradiation system 150 of this embodiment further includes a treatment plan unit 140.

The central control unit 100 includes a CPU 101 along with a memory 103 for storing information required to control the treatment, and cooperates with the accelerator controller 40 and the scanning controller 41.

The treatment plan information (patient information) of each of the patients is stored in the storage unit 110, and includes data such as an ID number, an irradiation dose (per treatment), irradiation energy, irradiation directions, and positions to be irradiated in the corresponding patient.

The scanning controller 41 is configured to control the scanning electromagnets 5A and 5B of the irradiation unit 15, and includes a memory 41A and a counter 41B. The memory 41A stores various types of treatment information, and the counter 41B is related to detection of the irradiation dose.

The counter 41B counts pulse signals outputted from the irradiation dose monitor 6A, so as to obtain the irradiation dose. This count of the pulse signals represents the irradiation dose measured from when the counting started. Hereinafter, the count of the pulse signals and the irradiation dose are considered as identical.

In the particle beam irradiation system 150 of this embodiment, based on the treatment plan information devised by the treatment plan unit 140, the central control unit 100, the scanning controller 41, and the accelerator controller 40 control operations in cooperation with each other.

The central control unit 100 reads the treatment plan information devised by the treatment plan unit 140 and stored by the storage unit 110, and then stores the treatment plan information in the memory 103.

Based on the treatment plan information stored in the memory 103, the CPU 101 generates information for the irradiation of the ion beam (information such as the number of a plurality of layers (the number of slices) in each area to be irradiated with the ion beam (the area including the affected area and the plurality of layers divided in depth direction), the number of positions (spots) to be irradiated, the positions to be irradiated in each of the slices, a target irradiation dose for each of the positions to be irradiated, and the information regarding all the spots in each of the slices, such as current values of the scanning electromagnets 5A and 5B). Then, the CPU 101 transmits the information above to the scanning controller 41. Here, the target irradiation dose for each of the positions to be irradiated with the ion beam may be a cumulative irradiation dose (integrated irradiation dose) measured from start of the irradiation at a first spot of the affected area, or may be an irradiation dose provided at each of the spots.

The CPU 101 selects, from the treatment plan information, all data for accelerator parameters of the synchrotron 12 with regard to all the slices, and transmits all the data to the accelerator controller 40.

Next, a control procedure executed by the scanning controller 41 and the accelerator controller 40 will be described.

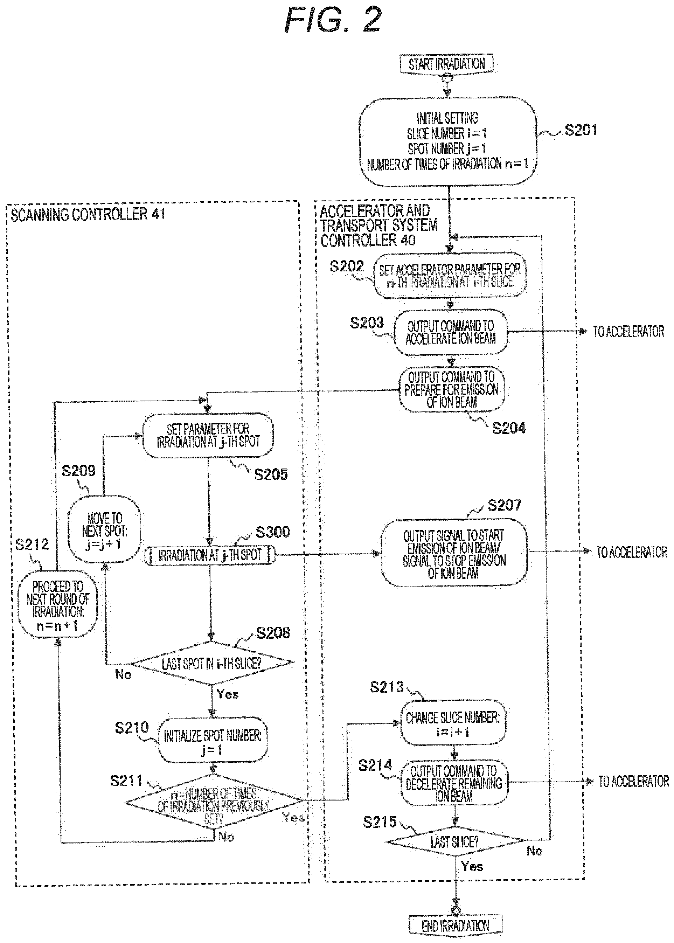

FIG. 2 is a flowchart of each of the scanning controller 41 and the accelerator controller 40, and shows how the scanning controller 41 and the accelerator controller 40 operate in cooperation during the irradiation.

When an irradiation start commander (not illustrated) in the treatment room is operated, the accelerator controller 40 in response initializes an operator "i" denoting a slice number to 1, an operator "j" denoting a spot number to 1, and "n" as the number of times of irradiation per slice to 1 in step S201.

Having completed initializing in the step S201, the accelerator controller 40 reads a plurality of the accelerator parameters stored in the memory 103 of the central control unit 100, and sets an accelerator parameter for an i-th (i = 1 in this state, i.e., first) slice in step S202; and the accelerator controller 40 outputs the accelerator parameter for the i-th slice to the synchrotron 12 in step S203.

In the step S203, the accelerator controller 40 outputs, to the power supply of each of the electromagnets in the synchrotron 12 and in the beam transport system 4, information included in the accelerator parameter for the i-th slice, the information for energizing current of the corresponding electromagnet. Based on the information for the energizing current, the accelerator controller 40 controls the power supply of each of the electromagnets such that the corresponding electromagnet is energized by the current predetermined.

Additionally, in the step S203, the accelerator controller 40 controls the radio-frequency power supply configured to apply the radio-frequency power to the radio-frequency accelerator cavity of the accelerator mechanism 10, so as to increase the radio-frequency power and a frequency wave up to values predetermined.

With this configuration, the energy of the ion beam circulating in the synchrotron 12 is increased to the value predetermined in the treatment plan. Subsequently, the accelerator controller 40 proceeds to step S204, and outputs to the scanning controller 41 a command to prepare for emission of the ion beam.

When the scanning controller 41 has received from the accelerator controller 40 information for the initialization in the step S201 and the command to prepare for the emission of the ion beam in the step S204, the scanning controller 41 reads data for current values and data for the target irradiation doses, the data stored in the memory 41A, to set the current value and the target irradiation dose for a j-th (j = 1 in this state, i.e., first) spot in step S205.

When the scanning controller 41 has been prepared for the irradiation at the j-th spot, in step S300, the scanning controller 41 outputs to the accelerator controller 40 a signal to start the emission of the ion beam. Then, the accelerator controller 40 controls the radio-frequency applicator 9 such that the ion beam is emitted from the synchrotron 12 through the emission deflector 8 to the beam transport system 4 (in step S207). In other words, in response to the signal to start the emission of the ion beam, the signal outputted from the scanning controller 41 (irradiation at the j-th spot in the step S300), the accelerator controller 40 operates the opening/closing switch 92 of the radio-frequency applicator 9 to close the opening/closing switch 92. As a result, the radio frequency power is applied from the radio-frequency power supply 91 to the radio-frequency applicator electrode 93.

Then, the radio-frequency power is applied from the radio-frequency applicator electrode 93 to the ion beam circulating within the stability limit in the synchrotron 12, thereby causing the energy of the ion beam to move out of the stability limit to be emitted from the synchrotron 12 through the emission deflector 8 to the beam transport system 4. The ion beam, which has been emitted from the synchrotron 12 to the beam transport system 4, travels through the beam path 62 to reach the irradiation unit 15.

On receipt of control signal from the scanning controller 41 in the step S300, the scanning electromagnets 5A and 5B of the irradiation unit 15 are energized such that the ion beam reaches the first spot. With this configuration, the irradiation unit 15 irradiates the first spot in the corresponding slice (i.e., first slice) with the ion beam.

When the irradiation dose at the first spot has reached the target irradiation dose for the first spot, and when the spot corresponds to a spot where the emission of the ion beam should stop, the scanning controller 41 outputs a signal to stop the emission of the ion beam (in the step S207), and subsequently proceeds to step S208.

When the first slice still has spots to be irradiated, the step S208 results in "no", and the scanning controller 41 thus proceeds to step S209 where "1" is added to the operator "j" denoting the spot number (in other words, a spot adjacent to the j-th spot is to be irradiated). Then, the scanning controller 41 repeats the steps S205, S300, and S208. In other words, until all the spots of the first slice have been irradiated with the ion beam, the scanning electromagnets 5A and 5B sequentially irradiates all the spots with the ion beam by moving the ion beam from one spot to another spot that is adjacent to the one spot (spot-by-spot scanning irradiation).

When all the spots of the first slice have been irradiated with the ion beam "n" = 1 times (i.e., once), the step S208 result in "yes". In this state, the scanning controller 41 initializes the operator "j" denoting the spot number (in step S210), and when "n" as the number of times of the irradiation set is not satisfied (in other words, all the spots of the first slice have not been irradiated with the ion beam at set number of times), step S211 results in "no". Then, the scanning controller 41 adds "1" to the "n" in step S212, and proceeds to the step S205 for a next round of irradiation.

On the other hand, when "n" as the number of times of the irradiation set is satisfied (in other words, all the spots of the first slice have been irradiated with the ion beam at the set number of times), the step S211 results in "yes". Then, the scanning controller 41 outputs to a CPU of the accelerator controller 40 a command to change the slice.

Having received the command to change the slice, the CPU of the accelerator controller 40 adds "1" to "i" denoting the slice number in step S213 (in other words, changing an object to be irradiated to a second slice), and outputs to the synchrotron 12 a command to decelerate the ion beam remaining in step S214.

The accelerator controller 40 outputs the command to decelerate the ion beam remaining, so as to control the power supply of each of the electromagnets of the synchrotron 12 to gradually reduce the energizing current for the corresponding electromagnet to a value predetermined, such as a value causing the energy of the ion beam to be circulating within the stability limit in the synchrotron 12. With this configuration, the ion beam circulating in the synchrotron 12 is decelerated.

The ion beam is emitted from the synchrotron 12 for a period of time that varies in accordance with the number of the spots within each of the slices and the irradiation dose. In this state, only the first slice has been irradiated with the ion beam, and thus when the second and subsequent slices are to be irradiated, step S215 results in "no". In this case, the accelerator controller 40 returns to the step S202 and reads from a memory of the accelerator controller 40 and sets the accelerator parameter for the i-th (i = 2) slice (second slice).

Subsequently, the steps S203 to S215 are executed for the second slice. Further, the steps S202 to S215 are to be executed until when all the spots in the final slice have been irradiated with the ion beam.

When the step S215 results in "yes" (all the spots of all the slices in the object to be irradiated, i.e., the patient 30, have been irradiated as predetermined), the CPU of the accelerator controller 40 outputs to the CPU 101 of the central control unit 100 the signal to end the irradiation.

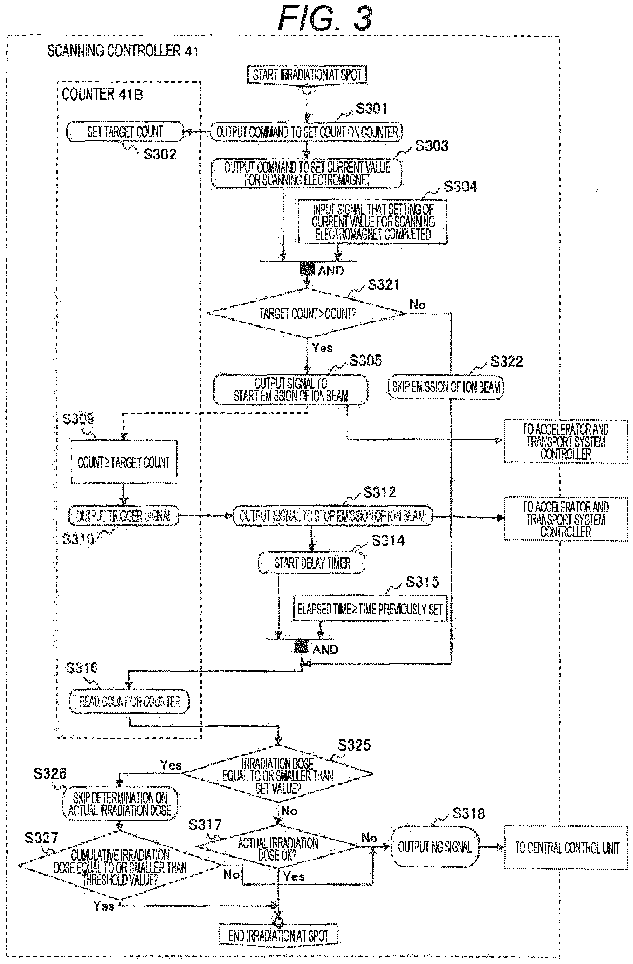

Next, the details of the control procedure executed by the scanning controller 41 and an example of an operation of a "skip-spot function" according to this embodiment will be described with reference to FIGS. 3 to 4.

FIG. 3 shows the details of the control procedure executed by the scanning controller 41 in the steps S205 and S300 of FIG. 2.

As a step corresponding to the step S205 of FIG. 2, in FIG. 3, the scanning controller 41 outputs to the counter 41B a command to set the count of the pulse signals, the count corresponding to the target irradiation dose that has been stored in the memory 41A, in step S301.

Based on the command, the counter 41B sets a target count for the j-th spot of the i-th slice in step S302.

When the step S301 has completed, the scanning controller 41 proceeds to step S303 as a step corresponding to the step S300 of FIG. 2, and outputs to a power supply of the scanning electromagnets 5A and 5B a command to set the current value for each of the scanning electromagnets 5A and 5B at the corresponding spot. Each of the scanning electromagnets 5A and 5B generates deflection electromagnetic force based on the current value above, and outputs to the scanning controller 41 a "current value setting complete" signal indicating that the scanning electromagnets 5A and 5B are in such a state (in step S304).

On conditions of having outputted the command to set the current values for the scanning electromagnets 5A and 5B (in the step S303) and having received the current value setting complete signal from the scanning electromagnets 5A and 5B (in the step S304), the scanning controller 41 proceeds to step S321 to determine whether or not to start the emission of the ion beam at the corresponding spot. Alternatively, without satisfying the condition of having received the current value setting complete signal (in the step S304), the scanning controller 41 may proceed to the step S321.

In the step S321, the scanning controller 41 determines, before outputting the signal to start the emission of the ion beam (step S305), whether or not the count on the counter 41B has exceeded the target count set in the step S302.

Here, when the target count is greater than the count on the counter 41B ("yes" in the step S321), the scanning controller 41 proceeds to the step S305. On the other hand, when the target count is equal to or smaller than the count on the counter 41B ("no" in the step S321), the corresponding spot has been irradiated with the ion beam of the target irradiation dose. Thus, the scanning controller 41 skips the emission of the ion beam at the corresponding spot (in step S322), and proceeds to step S316. As long as the step S321 results in "no", the scanning controller 41 skips the emission of the ion beam in the step S322. Thus, it is also possible to skip the emission of the ion beam at two or more consecutive spots.

Having proceeded to the step S322, the scanning controller 41 proceeds immediately to steps to complete the irradiation at the corresponding spot (i.e., the step S316 and subsequent steps). Accordingly, when the scanning controller 41 has proceeded to the step S321 without satisfying the condition of the step S304, as a condition to proceed to the step S322, the step S304 may be additionally included in the control procedure here. With this configuration, it is possible to avoid, after satisfying the condition of the step S304 at the corresponding spot, repeating the step S304 at a spot subsequent to the corresponding spot.

When the step S321 results in "yes", the scanning controller 41 outputs to the accelerator controller 40 the signal to start the emission of the ion beam in the step S305. On receipt of the signal to start the emission of the ion beam, the accelerator controller 40 controls the opening/closing switch 92 of the radio-frequency applicator 9 to close the opening/closing switch 92. Consequently, the radio-frequency power is applied from the radio-frequency power supply 91 to the radio-frequency applicator electrode 93.

The radio-frequency power applied to the radio-frequency applicator electrode 93 causes the energy of the ion beam circulating within the stability limit in the synchrotron 12 to move out of the stability limit, and then to be emitted from the synchrotron 12, through the emission deflector 8, to the beam transport system 4. Having been emitted to the beam transport system 4, the ion beam is transported through the beam path 62, and then is emitted from the irradiation unit 15 to the corresponding spot in the affected area of the patient 30 lying on the treatment bed 29.

When the count on the counter 41B, the count based on the pulse signals inputted to the scanning controller 41, is equal to or greater than a set value of the target count (target irradiation dose) that has been set in the step S302 (in step S309), the counter 41B outputs a trigger signal in step S310.

On receipt of the trigger signal, the scanning controller 41 generates the signal to stop the emission of the ion beam based on the trigger signal, and outputs to the accelerator controller 40 the signal to stop the emission of the ion beam (in step S312) . On receipt of the signal to stop the emission of the ion beam, the accelerator controller 40 controls the opening/closing switch 92 to open the opening/closing switch 92. In this state, the radio-frequency power, which is applied from the radio-frequency power supply 91 to the radio-frequency applicator electrode 93, is turned off. Consequently, the energy of the ion beam becomes within the stability limit, the emission of the ion beam from the synchrotron 12 stops, and the emission of the ion beam to the patient stops.

The scanning controller 41 includes a delay timer (not illustrated) that is configured to cause the scanning controller 41 to wait until the synchrotron 12 responds to the command from the accelerator controller 40 to stop the emission of the ion beam (wait until the emission of the ion beam completely stops). The trigger signal outputted in the step S310 is inputted as a command signal to start the delay timer (in step S314).

When time passed from start of the delay timer has reached delay time previously set on the delay timer, a "delay time is up" signal is outputted (in step S315).

On conditions of receiving the command signal to start the delay timer and outputting the "delay time is up" signal, in the step S316, the scanning controller 41 reads the count on the counter 41B, the count of the pulse signals outputted from the irradiation dose monitor 6A. Alternatively, instead of waiting until the delay time (previously set on the delay timer) is up, the system may use the electromagnets in the beam transport system 4 or others to stop the emission of the ion beam at the patient 30.

Next, the scanning controller 41 determines whether or not the irradiation dose at the corresponding spot is equal to or smaller than the set value (in step S325). When the irradiation dose is not equal to or smaller than the set value, the step S325 results in "no", and the scanning controller 41 proceeds to step S317.

In the step S317, in order to check whether or not an actual irradiation dose at the each of various spots in the patient 30 that has been irradiated with the ion beam is within a range predetermined, the scanning controller 41 determines on the actual irradiation dose based on the count that has been read. When the actual irradiation dose is determined as no good, the step S317 results in "no", and the scanning controller 41 outputs a no-good (NG) signal to the central control unit 100 (in step S318). On the other hand, when the actual irradiation dose is determined as good, the step S317 results in "yes". Then, the scanning controller 41 outputs the count as information of the actual irradiation dose to the central control unit 100, and ends the irradiation at the corresponding spot.

Here, some of the various spots require a small irradiation dose, so that calculation accuracy of the actual irradiation doses may be significantly degraded. Accordingly, each of the actual irradiation doses has the corresponding set value predetermined, and when the actual irradiation dose at the corresponding spot is equal to or smaller than the set value, the step S325 results in "yes". Then, the scanning controller 41 skips determination on the actual irradiation dose (in step S326), and ends the irradiation at the corresponding spot.

With this configuration, even at the spot requiring the small irradiation dose, it is possible to prevent detection of an error caused by the degraded calculation accuracy and thus possible to continue with the irradiation. With regard to the spot, at which the scanning controller 41 has skipped the emission of the ion beam (in the step S322), when the step S325 results in "yes", the scanning controller 41 skips the determination. Whenever each of the irradiation doses is equal to or smaller than the set value predetermined for the corresponding actual irradiation dose, the scanning controller 41 skips the determination. Accordingly, the scanning controller 41 may skip the determination consecutively at a plurality of the spots.

Here, in order to monitor the irradiation doses at the spots where the scanning controller 41 has skipped the determination, the scanning controller 41 records the actual irradiation dose at each of the spots where the scanning controller 41 has skipped the determination. Then, the scanning controller 41 compares the value (cumulative irradiation dose) that has been recorded with a threshold value that has been set before the irradiation (in step S327) . When the value (cumulative irradiation dose) is equal to or smaller than the threshold value ("yes" in the step S327), the scanning controller 41 determines that the cumulative irradiation dose is so small as not to affect the safety even when the treatment continues without the determination. Then, the scanning controller 41 completes the spot-by-spot irradiation and proceeds to the step S208 in FIG. 2.

On the other hand, when the value exceeds the threshold ("no" in the step S327), the scanning controller 41 determines that the cumulative irradiation dose suggests safety may be degraded when the treatment continues without the determination. Then, the scanning controller 41 outputs the NG correction signal (in the step S318) and does not proceed to the step S208. When the ion beam is being emitted then, the scanning controller 41 outputs the signal to stop the emission of the ion beam (in the step S207), and suspends the consecutive emission of the ion beam. Note that, when skipping the determination consecutively at the plurality of spots, the scanning controller 41 accumulates the irradiation doses at these plurality of spots to record as the cumulative irradiation dose.

With this function, it is possible to constantly monitor the irradiation doses at the spots where the scanning controller skips the determination, and thus possible to prevent the safety of the treatment from being degraded by skipping the determination. Here, instead of the irradiation doses, the number of times of skipping the determination may be monitored. The irradiation doses or the number of times of skipping the determination, each of which has been recorded, may be reset when the safety is confirmed.

Further, when the scanning controller 41 has skipped the emission of the ion beam (in the step S322), as has been described above, for the safety of the treatment, it is possible to monitor the number of times of skipping the emission as well as the irradiation dose at the spot where the scanning controller 41 has skipped the emission of the ion beam.

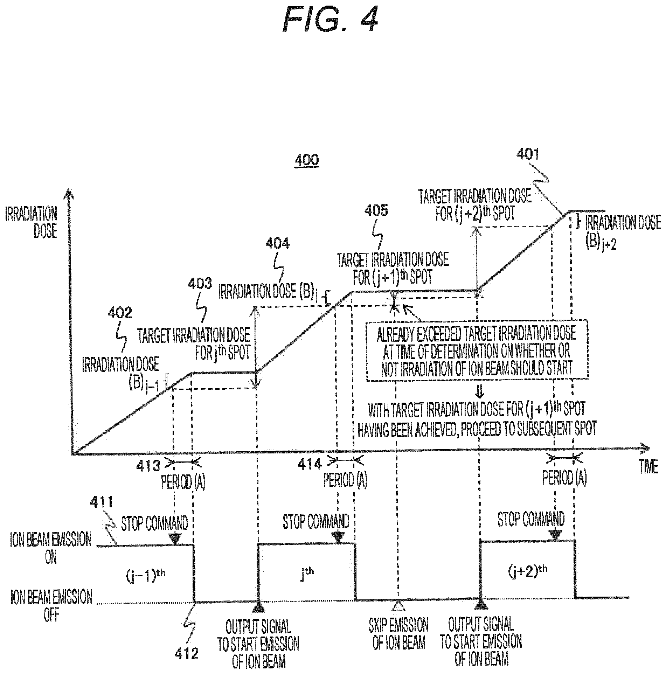

FIG. 4 shows a graph 400 as a timing chart of the irradiation dose when the irradiation is provided at four consecutive spots, such as a (j-1)th spot, j-th spot, a (j+1)th spot, and a (j+2)th spot. The graph 400 of FIG. 4 shows an example where a cumulative irradiation dose 401, which is measured from start of the irradiation at a first one of the consecutive spots, is monitored as an irradiation dose at each of the consecutive spots.

As has been described above, the delay may occur between when the scanning controller 41 outputs the signal to stop the emission of the ion beam and when the accelerator controller 40 operates the opening/closing switch 92 to actually stop the emission of the ion beam from the synchrotron 12. Meanwhile, during a period from when the signal (a stop command in FIG. 4) to stop the emission of the ion beam is outputted until when an ion-beam emission-on state 411 changes to an ion-beam emission-off state 412 (in FIG. 4) to cause the emission of the ion beam to actually stop, the period corresponding to a period (A): 413 in FIG. 4, the irradiation dose monitor 6A measures the irradiation dose of the ion beam emitted from the synchrotron 12. Then, the irradiation dose is to be accumulated at the counter 41B. The irradiation dose measured during the response delay of the accelerator is referred to as an irradiation dose (B)xx (xx corresponding to a spot number of the spot where the irradiation dose is measured during the response delay), such as an irradiation dose (B)j-1: 402. The irradiation dose (B)xx varies in accordance with the beam current value.

The relationship between proceeding from one of the spots to another one of the spots and the cumulative irradiation dose 401 in FIG. 4 will be specifically described with reference to the flowcharts of FIGS. 2 and 3.

When the emission of the ion beam has actually stopped, in the steps S209 and S205, the scanning controller 41 reads from the memory 41A the data for the current value and the data for the target irradiation dose of the position (spot) to be irradiated subsequent to the spot that has been irradiated (hereinafter, may also simply be referred to as a "subsequent position (spot) to be irradiated" or "subsequent position (spot)"). Then, in the step S301, the scanning controller 41 outputs to the counter 41B the command to set the count corresponding to the target irradiation dose. Subsequently, in the step S302, the counter 41B sets the target count for the subsequent position (spot) to be irradiated. In the step S303, the scanning controller 41 outputs the command to set the current value, based on which the scanning electromagnets 5A and 5B are controlled, so that the irradiation of the ion beam moves to the subsequent spot (position). In the step S305, the scanning controller 41 outputs the signal to start the emission of the ion beam, based on which the emission of the ion beam from the synchrotron 12 resumes.

In this state, the irradiation dose (count) at the subsequent spot includes, not only the irradiation dose measured by the irradiation dose monitor 6A from when the emission of the ion beam resumes, but also the irradiation dose (B)xx of FIG. 4.

For example, the irradiation dose at the j-th spot of FIG. 4 includes the irradiation dose (B)j-1: 402 measured at the (j-1)th spot during the period (A). Here, the irradiation dose (B)j-1: 402 corresponds to an initial value of the irradiation dose at the j-th spot, so that the irradiation dose at the j-th spot results in a cumulative value of the initial value and the irradiation dose measured from when the emission of the ion beam resumes. When the irradiation dose at the j-th spot has reached the target irradiation dose for the j-th spot (the irradiation dose shown with a "target irradiation dose for j-th spot 403" in FIG. 4), the scanning controller 41 outputs to the accelerator controller 40 the signal to stop the emission of the ion beam. Then, the accelerator controller 40 operates the opening/closing switch 92 to be open, so that the emission of the ion beam from the synchrotron 12 stops. Here, during a period from the output of the signal from the scanning controller 41 to stop the emission of the ion beam until the actual stop of the emission of the ion beam, the period corresponding to a period (A): 414 at the j-th spot, the j-th spot is irradiated with the ion beam of an irradiation dose (B)j: 404. The irradiation dose (B)j: 404 measured during the period (A) : 414 corresponds to an initial value of the irradiation dose at the (j+1)th spot subsequent to the j-th spot, and the irradiation dose at the (j+1)th spot corresponds to a cumulative value of the initial value and the irradiation dose measured from when the emission of the ion beam to the (j+1)th spot starts. Each of the subsequent spots undergoes repetition of the process above.

As has been described above, the scanning controller 41 executes the control procedure based on the irradiation dose. Thus, the irradiation of the ion beam is provided at each of the plurality of spots until a total value of the irradiation dose (B)xx measured at the spot prior to the corresponding spot during the response delay, i.e., the period (A), and the irradiation dose measured at the corresponding spot reaches the target irradiation dose for the corresponding spot.

Here, a spot requiring an extremely small target irradiation dose, such as the (j+1)th spot requiring a target irradiation dose for the (j+1)th spot 405 in FIG. 4, will be described.

In this case, when the j-th spot has been irradiated with the ion beam of the target irradiation dose for the j-th spot 403, and when the scanning controller 41 determines whether or not the target count corresponding to the target irradiation dose for the (j+1)th spot 405 is greater than the count corresponding to the irradiation dose (B)j: 404 of the ion beam measured at the j-th spot during the period (A) : 414 (in the step S321 to determine whether or not to output the signal to start the emission of the ion beam), the irradiation dose (B)j: 404 has already exceeded the target irradiation dose for the (j+1)th spot 405 (the count on the counter has already exceeded the target count for the (j+1)th spot). In this state, at the (j+1)th spot, the scanning controller 41 determines "no" in the step S321, and does not output the signal to start the emission of the ion beam but skips the (j+1)th spot (in the step S322).

The scanning controller 41 may include a function to monitor whether or not timing to skip is within a tolerance range immediately before the step S322, so that unexpected skipping of the spots is avoidable. Additionally, as has been described above, as the condition to proceed to the step S322, the step S304 may be additionally included in the control procedure at the (j+1)th spot.

Subsequently, the scanning controller 41 proceeds to the steps from when the emission of the ion beam has stopped (the step S316 and subsequent steps), and proceeds to the subsequent spot (in this case, the (j+2)th spot). With regard to the spot that the scanning controller 41 has skipped, the actual irradiation dose equals zero, and the step S325 described above results in "yes". Then, the scanning controller 41 skips the step S317 where the actual count on the counter is to be checked (and proceeds to the step S326).

As has been described above, with the function to skip the determination on the actual irradiation dose (hereinafter, referred to as a "skip function A") in the step S326 and the function to skip the irradiation of the ion beam (hereinafter, referred to as a "skip function B") in the step S322 at each of the spots, it is possible to irradiate any one of the spots requiring the small irradiation dose (spots where the target irradiation dose is zero or extremely small). These functions are collectively referred to as the "skip-spot function".

The particle beam irradiation system according to this embodiment, the particle beam irradiation system including the skip-spot function as has been described above, is effective as follows.

With the skip-spot function, even when, at the output of the signal to start the emission of the ion beam at each of the spots, the irradiation dose at the corresponding spot has exceeded the target irradiation dose for the corresponding spot, or even when the small irradiation dose is required in response to the response delay of the accelerator, complex shape, or complex irradiation dose distribution, it is possible, on the condition that sufficient safety is maintained, to continue with the treatment, and possible to prevent the occurrence of the errors causing the suspension of the irradiation and further continue with the treatment.

Even when the irradiation dose required at each of the spots is extremely small under various circumstances, or even when the irradiation dose (B)j-1: 402 or the irradiation dose (B)j: 404 of FIG. 4 unexpectedly increases, the skip-spot function is configured to prevent the suspension of the irradiation. Accordingly, the treatment plan does not necessarily include any particular arrangement for the spots requiring the small irradiation dose, so that the treatment plan becomes more efficient and quality of the treatment also improves.

Additionally, the lower limit of the irradiation dose at each of the spots, which imposes restriction on the treatment, is more relaxed, so that the treatment is more flexibly conducted (in response to the complex shape or irradiation dose distribution), without being required to achieve the irradiation dose at each single spot.

Further, as has been described above, together with the skip-spot function, at the spots where the skip function A is applied, the monitoring function is included to monitor the irradiation doses or the number of times of skipping the determination on the irradiation doses, so that the safety is secured. For further improvement of the safety, the monitoring function related to the skip function B may be included.

Note that, the spots where the skip-spot function is applied are estimated to be less than 1% of all the spots in each treatment. Accordingly, it is possible to include the skip-spot function and concurrently keep the influence on the treatment to a minimum, and thus possible to maintain the quality of the current treatment.

### Second embodiment

Next, a particle beam irradiation system according to a second embodiment of the present invention will be described. The particle beam irradiation system of this embodiment has the same configuration as that of the particle beam irradiation system 150 described in the first embodiment and thus, a description of the overall configuration thereof will be omitted as appropriate. Unlike the first embodiment including the method to control the start and stop of the emission of the ion beam at each of the spots, in this embodiment, the particle beam irradiation system has a feature configured to start and stop the emission of the ion beam at any timing. Thus, in this embodiment, a scanning controller 41 of a particle beam irradiation system 150 has a different control procedure from that of the scanning controller 41 in the first embodiment.

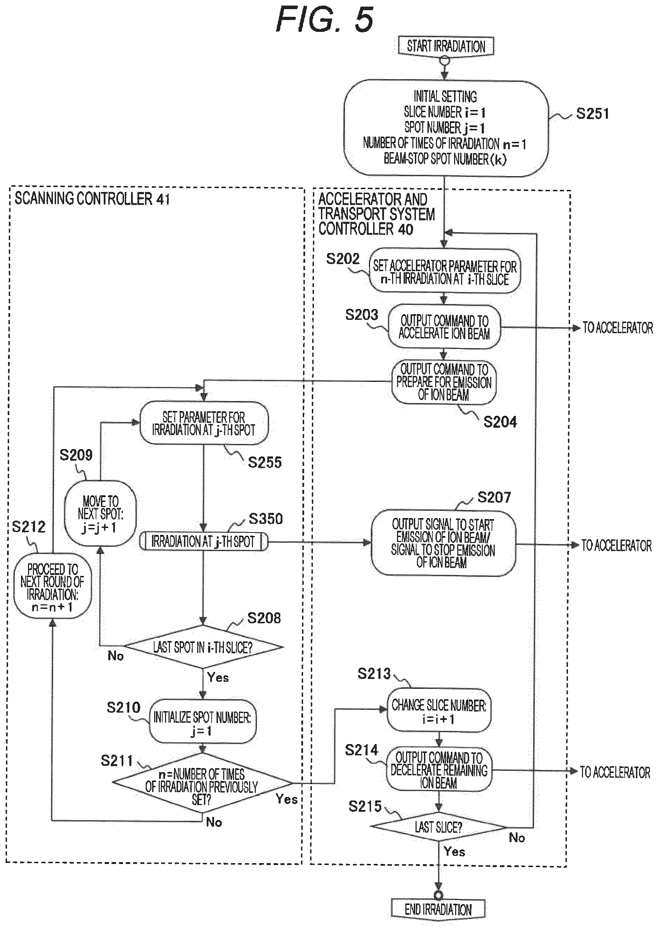

FIG. 5 is a flowchart showing the control procedure executed by each of the scanning controller 41 and an accelerator controller 40 of this embodiment, and shows how the scanning controller 41 and the accelerator controller 40 operate in cooperation. Each same step number represents the same process as in the flowchart of FIG. 2 of the first embodiment.

In step S251, as initial values to be set at the start of the irradiation of the ion beam, together with the items described in the first embodiment, a beam-stop spot number "k" is additionally included. In accordance with this setting, when the beam-stop spot number "k" has been irradiated, the emission of the ion beam stops. The beam-stop spot "k" may represent a plurality of spots.

Steps S255 and S350 respectively correspond to the steps S205 and S300 in the flowchart of FIG. 2 of the first embodiment. Additionally, other initial settings and a flow are the same as those in the flowchart of FIG. 2 of the first embodiment.

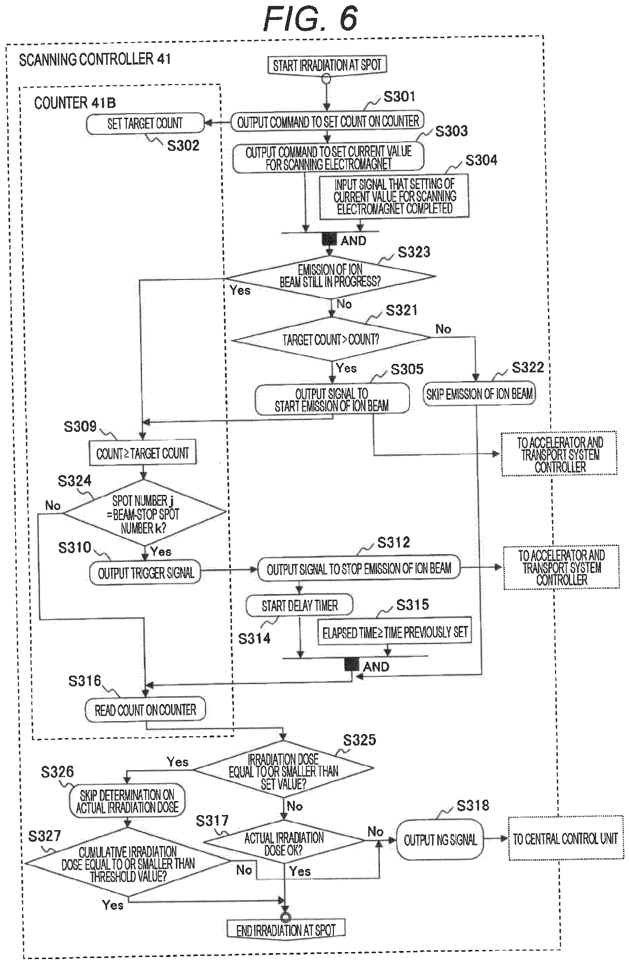

FIG. 6 shows the details of the control procedure executed by the scanning controller 41 of this embodiment in the steps S255 and S350 of FIG. 5.

As has been described above, in this embodiment, the particle beam irradiation system is configured to provide the irradiation of the ion beam at the plurality of spots without stopping the emission of the ion beam. Thus, at a first one of the plurality of spots where the emission of the ion beam starts, the same steps as in the first embodiment (i.e., the steps S301 to S304) are required to start the emission of the ion beam, but at each of the other spots where the emission of the ion beam has not stopped since the spot prior to the corresponding spot, the output of the signal to start the emission of the ion beam (step S305) is not required. In other words, in step S323 before the output of the signal to start the emission of the ion beam, the scanning controller 41 checks whether or not the emission of the ion beam is still in progress (whether or not the emission of the ion beam has never stopped since the spot prior to the corresponding spot). Then, when the step S323 results in "yes", the scanning controller 41 proceeds to step S309.

Subsequently, when the actual count is equal to or greater than the target count, the scanning controller 41 proceeds to step S324 to check whether or not the corresponding spot is a spot where the emission of the ion beam should stop. Specifically, the scanning controller 41 checks whether or not the spot number (i.e., "j") of the corresponding spot (i.e., the j-th spot) is identical to the beam-stop spot number "k". When the step S324 results in "yes", the scanning controller 41 proceeds to steps S310, S312, S314, and S315 to stop the emission of the ion beam as in the first embodiment.

Subsequently, when the signal to stop the emission of the ion beam has been outputted in the step S312 and time passed from the start of the delay timer in the step S314 has exceeded the delay time set previously in the step S315, the scanning controller 41 proceeds to step S316. In this embodiment too, the scanning controller 41 may use, instead of the delay timer, an electromagnet or others to stop the emission of the ion beam reaching the patient 30.

On the other hand, when the step S324 results in "no", the corresponding spot is not the stop where the emission of the ion beam should stop, and the scanning controller 41 proceeds to the step S316 without taking the steps to stop the emission of the ion beam. Here, the step S316 along with steps S317, S318, S325, S326, and S327 respectively correspond to the steps S316, S317, S318, S325, S326, and S327 of the first embodiment.

Meanwhile, when the emission of the ion beam has been suspended since the spot prior to the corresponding spot, the step S323 results in "no", and the scanning controller 41 proceeds to step S321 and subsequent steps to start the emission of the ion beam. Here, the steps S321, S305, and S322 as the steps to start the emission of the ion beam are the same processes as the steps S321, S305, and S322 of the first embodiment. Having proceeded to the step S322, the scanning controller 41 proceeds to the step S316, so that while the emission of the ion beam remains suspended, the signal to stop the emission of the ion beam is not outputted again.

The skip-spot function of this embodiment has a concept as will be described below.

As has been described in the first embodiment with reference to FIG. 4, the skip function B is applied only when the irradiation dose (B)xx measured at the spot prior to the corresponding spot during the response delay has exceeded the target irradiation dose for the corresponding spot. The irradiation dose (B)xx is measured, as has been described above, during the response delay of the accelerator. Thus, at the spot where the emission of the ion beam is not suspended, the irradiation dose (B)xx is zero.

With this configuration, in this embodiment, only when the emission of the ion beam stops at the spot prior to the corresponding spot and the emission of the ion beam resumes at the corresponding spot, the skip function B is applied. Thus, with regard to the other spots where the steps to resume the emission of the ion beam is not required (the step S323 results in "yes" in FIG. 6), the skip function B is not applied. Note that, whether the emission of the ion beam is suspended or resumed, the skip function A is applied to all the spots.

With regard to the spot where the skip function B is applied, similarly to the first embodiment, before outputting the signal to start the emission of the ion beam (in the step S305 of FIG. 6), the scanning controller 41 determines whether or not to skip the emission of the ion beam at the spot (in the step S321 of FIG. 6). On determination to skip, the scanning controller 41 proceeds to the step S322 and subsequently to the step S316 in FIG. 6.

When the scanning controller skips the determination on the actual irradiation dose in the step S326, similarly as has been described in the first embodiment, in order to monitor these actual irradiation doses, the scanning controller 41 records the actual irradiation doses at the spots where the scanning controller 41 has skipped the determination. Then, the scanning controller 41 compares the value (cumulative irradiation dose) that has been recorded with a threshold value that has been set before the irradiation (in the step S327). When the value (cumulative irradiation dose) is equal to or smaller than the threshold value ("yes" in the step S327), the scanning controller 41 determines that the cumulative irradiation dose is so small as not to affect the safety even when the treatment continues without the determination. Then, the scanning controller 41 completes the spot-by-spot irradiation and proceeds to step S208 in FIG. 5.

On the other hand, when the value exceeds the threshold ("no" in the step S327), the scanning controller 41 determines that the cumulative irradiation dose suggests safety may be degraded when the treatment continues without the determination. Then, the scanning controller 41 does not proceed to the step S208, but validates the function to suspend the consecutive emission of the ion beam (in the step S318). Note that, when skipping the determination consecutively at the plurality of spots, the scanning controller 41 accumulates the irradiation doses at these plurality of spots to record as the cumulative irradiation dose.

With this function, it is possible to constantly monitor the irradiation doses at the spots where the scanning controller skips the determination, and thus possible to prevent the safety of the treatment from being degraded by skipping the determination. Here, instead of the irradiation doses, the number of times of skipping the determination may be monitored. The irradiation doses or the number of times of skipping the determination, each of which has been recorded, may be reset when the safety is confirmed.

Further, when the scanning controller 41 has skipped the emission of the ion beam (in the step 5322), as has been described above, for the safety of the treatment, it is possible to monitor the number of times of skipping the emission as well as the irradiation dose at the spot where the scanning controller 41 has skipped the emission of the ion beam.

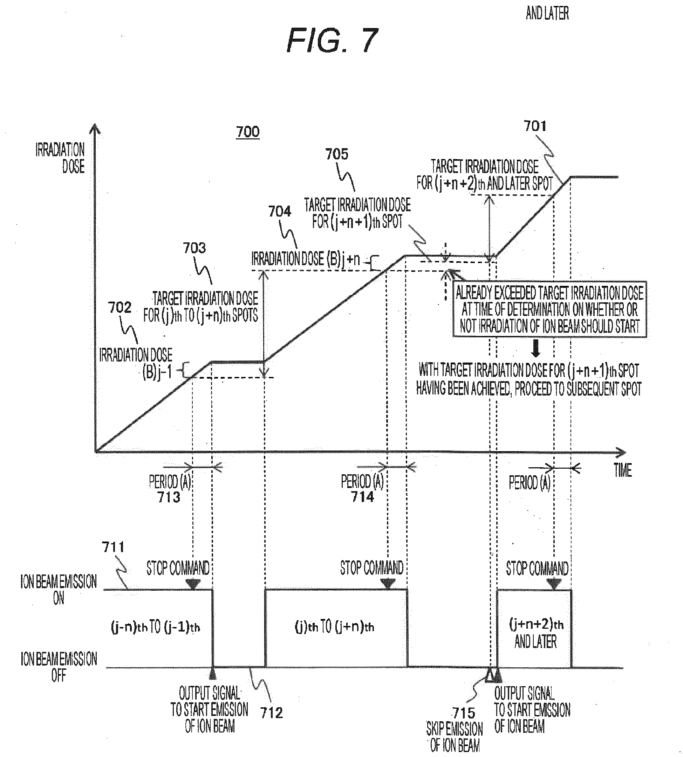

FIG. 7 shows a graph corresponding to the graph 400 of FIG. 4 that has been described in the first embodiment. In a case of the graph of FIG. 7, at a (j-n)th spot to a (j-1)th spot, at a j-th spot to a (j+n)th spot, at a (j+n+2)th and subsequent spots, an ion-beam emission-on state 711 is maintained, so that the emission of the ion beam is not suspended and the irradiation of the ion beam is consecutively provided at these spots; and between the (j-1)th spot and the j-th spot as well as between the (j+n)th spot and a (j+n+1)th spot, the ion beam is in an ion-beam emission-off state 712.

Similarly to FIG. 4, the graph of FIG. 7 shows an example where a cumulative irradiation dose 701, which is measured from start of the irradiation at a first one of the consecutive spots, is monitored as an irradiation dose at each of the consecutive spots.

Similarly as has been described in the first embodiment, the delay may occur between when the scanning controller 41 outputs the signal to stop the emission of the ion beam and when the accelerator controller 40 operates an opening/closing switch 92 to actually stop the emission of the ion beam from a synchrotron 12. Meanwhile, during a period from when the signal (a stop command in FIG. 7) to stop the emission of the ion beam is outputted until when the ion-beam emission-on state 711 changes to the ion-beam emission-off state 712 (in FIG. 7) to cause the emission of the ion beam to actually stop, the period corresponding to a period (A): 713 in FIG. 7, an irradiation dose monitor 6A measures the irradiation dose based on the ion beam emitted from the synchrotron 12. Then, the irradiation dose is to be accumulated at a counter 41B.

The irradiation dose measured during the response delay of the accelerator is referred to as an irradiation dose (B)xx (xx corresponding to a spot number of the spot where the irradiation dose is measured during the response delay), such as an irradiation dose (B)j-1: 702. The irradiation dose (B)xx varies in accordance with the beam current value.

The relationship between proceeding from one of the spots to another one of the spots and the cumulative irradiation dose 701 in FIG. 7 will be specifically described with reference to the flowcharts of FIGS. 5 and 6.

When the consecutive emission of the ion beam has actually stopped, in steps S209 and S205, the scanning controller 41 reads from a memory 41A the data for the current value and the data for the target irradiation dose of the position (spot) to be irradiated subsequent to the spot that has been irradiated (hereinafter, may also simply be referred to as a "subsequent position (spot) to be irradiated" or subsequent position (spot)"). Then, in the step S301, the scanning controller 41 outputs to the counter 41B the command to set the count corresponding to the target irradiation dose. Subsequently, in the step S302, the counter 41B sets the target count for the subsequent position (spot) to be irradiated. In the step S303, the scanning controller 41 outputs the command to set the current value, based on which the scanning electromagnets 5A and 5B are controlled, so that the irradiation of the ion beam moves to the subsequent spot (position). In the step S305, the scanning controller 41 outputs the signal to start the emission of the ion beam, based on which the emission of the ion beam from the synchrotron 12 resumes.

In this state, the irradiation dose (count) at the spot (where the irradiation resumes) includes, not only the irradiation dose measured by the irradiation dose monitor 6A from when the emission of the ion beam resumes, but also the irradiation dose (B)xx of FIG. 7.

For example, the irradiation dose at the j-th spot of FIG. 7 includes the irradiation dose (B)j-1: 702 measured during the period (A): 713 as a final part of the irradiation at the (j-n)th to (j-1)th spots. Here, the irradiation dose (B)j-1: 702 corresponds to an initial value of the irradiation dose at the j-th spot, so that the irradiation dose at the j-th spot results in a cumulative value of the initial value and the irradiation dose measured from when the emission of the ion beam resumes.

Then, at the j-th to (j+n)th spots where the irradiation is subsequently and consecutively provided, when the irradiation dose at each of these spots has reached the target count for the corresponding spot (set in the step S302), the scanning controller 41 determines in the step S324 whether or not the spot number of the corresponding spot is identical to the beam-stop spot number "k" set in the step S251.

When the scanning controller 41 determines that the spot number is identical to the beam-stop spot number "k" ("yes" in the step S324), the counter 41B outputs to the scanning controller 41 a trigger signal in the step S310. On receipt of the trigger signal, the scanning controller 41 transmits to the accelerator controller 40 the signal to stop the emission of the ion beam in the step S312. Then, the accelerator controller 40 operates the opening/closing switch 92 to be open, so that the emission of the ion beam from the synchrotron 12 stops.

In the example of FIG. 7, at the j-th to (j+n-1)th spots, the step S324 results in "no". The scanning controller 41 does not operate to stop the emission of the ion beam but proceeds to the step S316, and then proceeds to the irradiation at the spot (i.e., (j+n)th spot) subsequent to the (j+n-1)th spot.

Meanwhile, when the irradiation dose has reached the target count at the (j+n)th spot, in other words, when the irradiation dose has reached a target cumulative irradiation dose for a period from the j-th spot where the emission of the ion beam started until the (j+n)th spot (the irradiation dose shown as a "target irradiation dose for j-th to (j+n)th spots 703" in FIG. 7), the step S324 is determined as "yes".

Here, during a period from the output (from the scanning controller 41) of the signal to stop the emission of the ion beam until the actual stop of the irradiation of the ion beam, in other words, during a period (A): 714 as a final part of the irradiation at the j-th to (j+n)th spots, the spot (that is subjected to the final part of the irradiation) is irradiated with the ion beam of an irradiation dose (B)j+n: 704. The irradiation dose (B)j+n: 704 measured during the period (A): 714 corresponds to an initial value of the irradiation dose at the (j+n+1)th spot and subsequent spots, and the irradiation dose at the (j+n+1)th spot results in a cumulative value of the initial value and the irradiation dose measured from when the emission of the ion beam at the (j+n+1)th spot starts. The subsequent plurality of spots undergo repetition of the process above.

As has been described above, the scanning controller 41 executes the control procedure based on the irradiation dose. Thus, the emission of the ion beam is provided at each of the consecutive spots until the total value of the irradiation dose (B)xx measured at the spot prior to the consecutive spots during the response delay, i.e., the period (A), and the irradiation doses measured at the consecutive spots reaches the target irradiation dose for the consecutive spots.

Here, a spot having an extremely small target irradiation dose will be described, with reference, as an example, to the (j+n+1)th spot of FIG. 7 as a first spot where the irradiation resumes and where a target irradiation dose for the (j+n+1)th spot 705 is extremely small.

In this case, when the j-th to (j+n)th spots have been consecutively irradiated with the ion beam of the target irradiation dose for the j-th to (j+n)th spots 703, and when the scanning controller 41 determines whether or not, at the (j+n+1)th spot as the first spot of the subsequent consecutive irradiation, the target count corresponding to the target irradiation dose for the (j+n+1)th spot 705 is greater than the count corresponding to the irradiation dose (B)j+n: 704 measured during the period (A): 714 (in the step S321 to determine whether or not to output the signal to start the emission of the ion beam), the irradiation dose (B)j+n: 704 has already exceeded the target irradiation dose for the (j+n+1)th spot 705 (the count on the counter has already exceeded the target count for the (j+n+1)th spot). In this state, at the (j+n+1)th spot, the scanning controller 41 determines "no" in the step S321, and does not output the signal to start the emission of the ion beam but skips the (j+n+1)th spot (in the step S322).

The scanning controller 41 may include a function to monitor whether or not timing to skip is within a tolerance range immediately before the step S322, so that unexpected skipping of the spots is avoidable. Additionally, as has been described above, as the condition to proceed to the step S322, the step S304 may be additionally included in the control procedure at the (j+n+1)th spot and consecutively subsequent spots.

Subsequently, the scanning controller 41 proceeds to the steps required from when the emission of the ion beam has stopped (the step S316 and subsequent steps), and proceeds to a consecutively subsequent spot (in this case, the (j+n+2)th spot). With regard to the spot that the scanning controller 41 has skipped, the actual irradiation dose equals zero, and the step S325 described above results in "yes". Then, the scanning controller 41 skips the step S317 where the actual count on the counter is to be checked, and proceeds to the step S326.

As has been described above, similarly to the first embodiment, with the function to skip the determination on the actual irradiation dose (hereinafter, referred to as the "skip function A") in the step S326 and the function to skip the irradiation of the ion beam (hereinafter, referred to as the "skip function B") in the step S322 at each of the spots, it is possible to irradiate any one of the spots requiring the small irradiation dose (spots where the target irradiation dose is zero or extremely small). These functions are collectively referred to as the "skip-spot function".

As has been described with reference to FIG. 7, the skip function B is applied only when the irradiation dose (B)xx measured at the spot prior to the corresponding spot during the response delay has exceeded the target irradiation dose at the corresponding spot. The irradiation dose (B)xx is measured, as has been described above, during the response delay of the accelerator. Thus, at the spot where the emission of the ion beam is not suspended, the irradiation dose (B)xx is, zero.

With this configuration, in this embodiment, only when the emission of the ion beam stops at the spot prior to the corresponding spot and the emission of the ion beam resumes at the corresponding spot, the skip function B is applied. Thus, with regard to the other spots where the steps to resume the emission of the ion beam is not required (the step S323 results in "yes" in FIG. 6), the skip function B is not applied. Note that, whether the emission of the ion beam is suspended or resumed, the skip function A is applied to all the spots.

With regard to the spot where the skip function B is applied, similarly to the first embodiment, before outputting the signal to start the emission of the ion beam (in the step S305 of FIG. 6), the scanning controller 41 determines whether or not to skip the emission of the ion beam at the spot (in the step S321 of FIG. 6). On determination to skip, the scanning controller 41 proceeds to the step S322 and subsequently to the step S324 in FIG. 6.

As has been described above, the particle beam irradiation system according to this embodiment includes the skip-spot function, and is as effective as in the first embodiment. With the skip-spot function, even when, at the output of the signal to start the emission of the ion beam at each of the spots, the irradiation dose for the corresponding spot has exceeded the target irradiation dose for the corresponding spot, or even when the small irradiation dose is required in response to the response delay of the accelerator, complex shape, or complex irradiation dose distribution, it is possible, on the condition that sufficient safety is maintained, to continue with the treatment, and possible to prevent the occurrence of the errors causing the suspension of the irradiation and further continue with the treatment.

Even when the irradiation dose required at each of the spots is extremely small under various circumstances, or when the irradiation dose (B)j-1: 702 or the irradiation dose (B)j+n: 704 of FIG. 7 unexpectedly increases, the skip-spot function is configured to prevent the suspension of the irradiation. Accordingly, the treatment plan does not necessarily include any particular arrangement for the spots requiring the small irradiation dose, so that the treatment plan becomes more efficient and quality of the treatment also improves.

Additionally, the lower limit of the irradiation dose at each of the spots, which imposes restriction on the treatment, is more relaxed, so that the- treatment is more flexibly conducted (in response to the complex shape or irradiation dose distribution), without being required to achieve the irradiation dose at each single spot.

Further, as has been described above, together with the skip-spot function, at the spots where the skip function A is applied, the monitoring function is included to monitor the irradiation doses or the number of times of skipping the determination on the irradiation doses, so that the safety is secured. For further improvement of the safety, the monitoring function related to the skip function B may be included.

Note that, the spots where the skip-spot function is applied are estimated to be less than 1% of all the spots in each treatment. Accordingly, it is possible to include the skip-spot function and concurrently keep the influence on the treatment to a minimum, and thus possible to maintain the quality of the current treatment.

### Reference Signs List

1 charged particle beam generation unit
4 beam transport system
5A, 5B scanning electromagnet (charged particle beam scanner)
6A irradiation dose monitor (irradiation dose detector)
6B position monitor
8 emission deflector
9 radio-frequency applicator
10 accelerator mechanism
11 pre-stage charged particle beam generation unit (LINAC)
12 synchrotron (accelerator)
15 irradiation unit
17, 23, 24 deflection electromagnet
18, 21, 22 quadrupole electromagnet
29 treatment bed
30 patient
40 accelerator and transport system controller
41 scanning controller
41A memory
41B counter
62 beam path
91 radio-frequency power supply
92 opening/closing switch
93 radio-frequency applicator electrode
100 central control unit
140 treatment planning device

## Claims

1. A particle beam irradiation system comprising:
a charged particle beam generation unit configured to generate a charged particle beam;
an irradiation unit including a scanning electromagnet configured to provide spot-by-spot irradiation at an object to be irradiated with the charged particle beam generated by the charged particle beam generation unit, the spot-by-spot irradiation performed by sequentially providing irradiation of the charged particle beam at a plurality of spots one by one in the object, and an irradiation dose monitor configured to measure an irradiation dose of the charged particle beam;
a scanning controller configured to generate a signal to start or stop the irradiation of the charged particle beam provided by the irradiation unit at the object to be irradiated; and
an accelerator and transport system controller configured, on receipt of the signal to start or stop the irradiation of the charged particle beam outputted from the scanning controller, to start or stop emission of the charged particle beam from the charged particle beam generation unit to the irradiation unit,
wherein
when, on the receipt of the signal to stop the irradiation of the charged particle beam, the accelerator and transport system controller stops the emission of the charged particle beam from the charged particle beam generation unit to the irradiation unit, the scanning controller determines, in accordance with the irradiation dose at one of the plurality of spots that has been irradiated with the charged particle beam until immediately before the accelerator and transport system controller stops the emission, the irradiation dose measured by the irradiation dose monitor from when the signal to stop the irradiation is outputted from the scanning controller, whether or not to skip the irradiation of the charged particle beam at another one of the plurality of spots subsequent to the one of the plurality of spots, so as to control the accelerator and transport system controller.

2. A particle beam irradiation system comprising:
a charged particle beam generation unit configured to generate a charged particle beam;
an irradiation unit including a scanning electromagnet configured to provide spot-by-spot irradiation at an object to be irradiated with the charged particle beam generated by the charged particle beam generation unit, the spot-by-spot irradiation performed by sequentially and consecutively providing irradiation of the charged particle beam at a plurality of spots in the object, and an irradiation dose monitor configured to measure an irradiation dose of the charged particle beam;
a scanning controller configured to generate a signal to start or stop the irradiation of the charged particle beam provided by the irradiation unit at the object to be irradiated; and
an accelerator and transport system controller configured, on receipt of the signal to start or stop the irradiation of the charged particle beam outputted from the scanning controller, to start or stop emission of the charged particle beam from the charged particle beam generation unit to the irradiation unit,
wherein
the scanning controller controls the accelerator and transport system controller to consecutively provide the irradiation of the charged particle beam at the plurality of spots,
the scanning controller outputs to the accelerator and transport system controller the signal to stop the irradiation of the charged particle beam when a cumulative total of the irradiation dose of the charged particle beam consecutively provided at each of the plurality of spots, the irradiation dose measured by the irradiation dose monitor, has reached a predetermined irradiation dose, and
the scanning controller determines, in accordance with the irradiation dose measured by the irradiation dose monitor from when the signal to stop the irradiation is outputted, whether or not to skip the irradiation of the charged particle beam at a first one of a plurality of spots that are to be consecutively irradiated subsequent to the plurality of spots that have been consecutively irradiated with the charged particle beam, so as to control the accelerator and transport system controller.

3. The particle beam irradiation system according to claim 1 or 2, wherein
when the irradiation dose measured by the irradiation dose monitor at each of the plurality of spots that has been irradiated with the charged particle beam is greater than an irradiation dose previously set, the scanning controller determines whether the irradiation dose measured is normal or abnormal, and
when the irradiation dose measured at each of the plurality of spots is smaller than the irradiation dose previously set, the scanning controller skips determining whether the irradiation dose measured is normal or abnormal.

4. The particle beam irradiation system according to claim 3, wherein
the scanning controller compares a cumulative total of the irradiation dose measured at each of the plurality of spots, where the scanning controller has skipped determining whether the irradiation dose measured is normal or abnormal, with a threshold value previously set, and
when the cumulative total of the irradiation dose is greater than the threshold value, the scanning controller controls the accelerator and transport system controller to suspend the irradiation of the charged particle beam.

5. A control method for a particle beam irradiation system including a charged particle beam generation unit configured to generate a charged particle beam, an irradiation unit configured to sequentially provide irradiation of the charged particle beam at a plurality of spots one by one in an object to be irradiated, the irradiation unit including an irradiation dose monitor to measure an irradiation dose of the charged particle beam at the plurality of spots, a scanning controller configured to output a signal to start or stop the irradiation of the charged particle beam, and an accelerator and transport system controller configured, based on the signal outputted from the scanning controller, to start or stop the irradiation of the charged particle beam,
the control method, performed by the scanning controller, comprising:
outputting to the accelerator and transport system controller the signal to stop the irradiation of the charged particle beam when the irradiation dose of the charged particle beam at one of the plurality of spots that has been irradiated with the charged particle beam, the irradiation dose measured by the irradiation dose monitor, reaches a predetermined irradiation dose; and
determining, in accordance with the irradiation dose measured at the one of the plurality of spots by the irradiation dose monitor from when the signal to stop the irradiation is outputted, whether or not to skip the irradiation of the charged particle beam at another one of the plurality of spots subsequent to the one of the plurality of spots, so as to control the accelerator and transport system controller.

6. A control method for a particle beam irradiation system including a charged particle beam generation unit configured to generate a charged particle beam, an irradiation unit configured to consecutively provide irradiation of the charged particle beam at a plurality of spots in an object to be irradiated, the irradiation unit including an irradiation dose monitor to measure an irradiation dose of the charged particle beam at the plurality of spots, a scanning controller configured to output a signal to start or stop the irradiation of the charged particle beam, and an accelerator and transport system controller configured, based on the signal outputted from the scanning controller, to start or stop the irradiation of the charged particle beam,
the control method, performed by the scanning controller, comprising:
outputting to the accelerator and transport system controller the signal to stop the irradiation of the charged particle beam when a cumulative total of the irradiation dose of the charged particle beam consecutively provided at each of the plurality of spots, the irradiation dose measured by the irradiation dose monitor, reaches a predetermined irradiation dose, and
determining, in accordance with the irradiation dose measured by the irradiation dose monitor from when the signal to stop the irradiation is outputted, whether or not to skip the irradiation of the charged particle beam at a first one of a plurality of spots that are to be consecutively irradiated subsequent to the plurality of spots that have been consecutively irradiated with the charged particle beam, so as to control the accelerator and transport system controller.

7. The control method for the particle beam irradiation system according to claim 5 or 6, the control method, performed by the scanning controller, further comprising:
when the irradiation dose measured by the irradiation dose monitor at each of the plurality of spots that has been irradiated with the charged particle beam is greater than an irradiation dose previously set, determining whether the irradiation dose measured is normal or abnormal; and
when the irradiation dose measured at each of the plurality of spots is smaller than the irradiation dose previously set, skipping determining whether the irradiation dose -measured is normal or abnormal.

8. The control method for the particle beam irradiation system according to claim 7, the control method, performed by the scanning controller, further comprising:
comparing a cumulative total of the irradiation dose measured at each of the plurality of spots, where determining whether the irradiation dose measured is normal or abnormal has been skipped, with a threshold value previously set; and
when the cumulative total of the irradiation dose is greater than the threshold value, controlling the accelerator and transport system controller to suspend the irradiation of the charged particle beam.

9. A control device for a particle beam irradiation system including a charged particle beam generation unit configured to generate a charged particle beam, an irradiation unit configured to provide spot-by-spot irradiation at an object to be irradiated with the charged particle beam generated by the charged particle beam generation unit, the spot-by-spot irradiation performed by sequentially providing irradiation of the charged particle beam at a plurality of spots one by one in the object, and an irradiation dose monitor configured to monitor and measure an irradiation dose of the charged particle beam provided by the irradiation unit,
the control device comprising:
a scanning controller configured to generate a signal to start or stop the irradiation of the charged particle beam provided by the irradiation unit at the object to be irradiated; and
an accelerator and transport system controller configured, on receipt of the signal to stop the irradiation of the charged particle beam outputted from the scanning controller, to start or stop emission of the charged particle beam from the charged particle beam generation unit to the irradiation unit,
wherein
when an irradiation dose of the charged particle beam at one of the plurality of spots that has been irradiated with the charged particle beam, the irradiation dose measured by the irradiation dose monitor, has reached a predetermined irradiation dose, the scanning controller outputs to the accelerator and transport system controller the signal to stop the irradiation of the charged particle beam, and
the scanning controller determines,in accordance with the irradiation dose measured by the irradiation dose monitor at the one of the plurality of spots from when the signal to stop the irradiation is outputted, whether or not to skip the irradiation of the charged particle beam at another one of the plurality of spots subsequent to the one of the plurality of spots, so as to control the accelerator and transport system controller.

10. A control device for a particle beam irradiation system including a charged particle beam generation unit configured to generate a charged particle beam, an irradiation unit configured to consecutively provide irradiation of the charged particle beam generated by the charged particle beam generation unit at a plurality of spots in an object to be irradiated, and an irradiation dose monitor configured to monitor and measure an irradiation dose of the charged particle beam provided by the irradiation unit,
the control device comprising:
a scanning controller configured to generate a signal to start or stop the irradiation of the charged particle beam provided by the irradiation unit at the object to be irradiated; and
an accelerator and transport system controller configured, on receipt of the signal to stop the irradiation of the charged particle beam outputted from the scanning controller, to start or stop emission of the charged particle beam from the charged particle beam generation unit to the irradiation unit,
wherein
the scanning controller outputs to the accelerator and transport system controller the signal to stop the irradiation of the charged particle beam when a cumulative total of the irradiation dose of the charged particle beam consecutively provided at each of the plurality of spots, the irradiation dose measured by the irradiation dose monitor, has reached a predetermined irradiation dose, and
the scanning controller determines, in accordance with the irradiation dose measured by the irradiation dose monitor from when the signal to stop the irradiation is outputted, whether or not to skip the irradiation of the charged particle beam at a first one of a plurality of spots that are to be consecutively irradiated subsequent to the plurality of spots that have been consecutively irradiated with the charged particle beam, so as to control the accelerator and transport system controller.

11. The control device for the particle beam irradiation system according to claim 9 or 10, wherein
when the irradiation dose measured by the irradiation dose monitor at each of the plurality of spots that has been irradiated with the charged particle beam is greater than an irradiation dose previously set, the scanning controller determines whether the irradiation dose measured is normal or abnormal, and
when the irradiation dose measured at each of the plurality of spots is smaller than the irradiation dose previously set, the scanning controller skips determining whether the irradiation dose measured is normal or abnormal.

12. The control device for the particle beam irradiation system according to claim 11, wherein
the scanning controller compares a cumulative total of the irradiation dose measured at each of the plurality of spots, where the scanning controller has skipped determining whether the irradiation dose measured is normal or abnormal, with a threshold value previously set, and
when the cumulative total of the irradiation dose is greater than the threshold value, the scanning controller controls the accelerator and transport system controller to suspend the irradiation of the charged particle beam.
